# EUROPÄISCHE PATENTSCHRIFT

(11) **EP 1 780 195 B1**
(45) Veröffentlichungstag und Bekanntmachung des Hinweises auf die Patenterteilung: **22.07.2009**
(21) Anmeldenummer: 06019445.3
(22) Anmeldetag: 18.09.2006
(51) Int. Cl.: C07C 201/08, C07C 205/06

(54) **Rückgewinnung von Nitriersäuregemischen aus Nitrierprozessen**
Recovery of the nitration acid mixtures from nitration processes
Récupération de mélanges d'acides nitreux dans les procédés de nitration

(30) Priorität: 18.10.2005 DE 102005050106
(43) Veröffentlichungstag der Anmeldung: 02.05.2007
(73) Patentinhaber: Josef Meissner GmbH & Co. KG, 50968 Köln (DE)
(72) Erfinder: Hermann, Heinrich, Dr., 50858 Köln (DE); Gebauer, Jürgen, 53840 Troisdorf (DE); Konieczny, Peter, Dr., 14513 Teltow (DE); Händel, Mirko, Dr., 53819 Neunkirchen-Seelscheid (DE)
(74) Vertreter: Gesthuysen, von Rohr & Eggert

(56) Entgegenhaltungen:
- EP-A1- 0 736 514
- US-A- 4 257 986

## Beschreibung

Die vorliegende Erfindung betrifft die Entfernung und Rückgewinnung von Nitriersäuregemischen, insbesondere Gemischen von Schwefelsäure, Salpetersäure und Stickoxiden, aus Nitrierprozessen. Insbesondere betrifft die vorliegende Erfindung ein Verfahren zur Entfernung und Rückgewinnung von Nitriersäuregemischen, insbesondere Gemischen von Schwefelsäure, Salpetersäure und Stickoxiden, die bei der Nitrierung von nitrierbaren aromatischen Verbindungen (z. B. Benzol, Toluol, Chlorbenzol, Dichlorbenzole, Mononitrobenzol, Mononitrochlorbenzole, Mononitrotoluole, Dinitrotoluole etc.) nach Abtrennung der Nitriersäure in den nitrierten Rohprodukten anfallen. Gemäß einem besonderen Aspekt betrifft die vorliegende Erfindung ein Verfahren zur Entfernung und Rückgewinnung von Nitriersäuregemischen, insbesondere Gemischen von Schwefelsäure, Salpetersäure und Stickoxiden, aus den bei der Nitrierung von Toluol oder Mononitrotoluolen (MNT) nach Abtrennung der Nitriersäure anfallenden rohen Dinitrotoluole (DNT). Des weiteren betrifft die vorliegende Erfindung eine Anlage zur Nitrierung nitrierbarer aromatischer Verbindungen mit nachfolgender Aufreinigung der nitrierten Produkte einschließlich einer Entfernung und Wiedergewinnung von Nitriersäuregemischen, insbesondere Schwefelsäure, Salpetersäure und Stickoxiden

Die Nitrierung insbesondere aromatischer Verbindungen ist eine der prominentesten Reaktion in der organischen Chemie, da sie von großer technischer Bedeutung ist und daher universell eingesetzt wird, so z. B. bei der Herstellung von Explosivstoffen, in der pharmazeutischen Industrie, bei der Herstellung von Kunststoffen, bei der Produktion von Farbstoffen und dergleichen. Für die präparative Chemie von Bedeutung ist vor allem die elektrophile Nitrierung aromatischer Verbindungen, und zwar zum einen, weil fast alle Aromaten mit einem geeigneten Nitrierungsreagenz nitriert werden können, und zum anderen, weil die aromatische Nitrogruppe leicht in andere funktionelle Gruppen umgesetzt werden kann. Das einfachste und technisch am häufigsten eingesetzte Nitrierungsreagenz sind Salpetersäure/Schwefelsäure-Gemische (sogenannte "Nitriersäure") mit variablen Mengenverhältnissen der beiden Säuren. Dabei bewirkt die konzentrierte Schwefelsäure die Bildung des eigentlich wirksamen Nitrylkations und bindet daneben das beim Nitrierprozeß entstehende Wasser. Für weitere Einzelheiten zur Nitrierung kann beispielsweise auf Römpp Chemielexikon, 10. Auflage, Georg Thieme Verlag, Stuttgart/New York, Band 4, 1998, Seiten 2914/2915, Stichworte: "Nitrierung" und "Nitriersäure", sowie die dort jeweils referierte Literatur verwiesen werden.

Bei technischen Prozessen werden erhebliche Mengen an Nitriersäure eingesetzt. Da die Nitriersäure im allgemeinen in relativ großem Überschuß eingesetzt wird, besteht aus Rentabilitätsgründen die Zielsetzung nicht nur darin, die überschüssige Nitriersäure einschließlich der gebildeten Stickoxide aus den nitrierten Produkten zu entfernen bzw. hiervon möglichst vollständig abzutrennen, um hochreine, insbesondere säurefreie Nitrierprodukte zu erhalten, sondern auch darin, die abgetrennte überschüssige Nitriersäure einschließlich der gebildeten Stickoxide auch wieder für den Nitrierprozeß nutzbar zu machen und mit möglichst geringem Aufwand zu rezyklieren. Auch aus ökologischen Gründen, insbesondere auch im Hinblick auf diesbezügliche gesetzliche Vorschriften, ist es erforderlich, die eingesetzte überschüssige Nitriersäure möglichst vollständig zu entfernen und wiederzugewinnen, damit die Belastung der aus dem Nitrierprozeß stammenden Abwässer, insbesondere die Nitratfracht, auf ein Minimum reduziert werden kann.

Aufgrund der Komplexität der zugrundeliegenden physikochemischen Gegebenheiten ist die Lösung dieses Problems aber nicht trivial, und es hat im Stand der Technik bereits zahlreiche Ansätze und Versuche zur Lösung dieses Problems gegeben, wobei noch keine umfänglich zufriedenstellende Lösung aufgezeigt worden ist.

Die Komplexität des zuvor geschilderten Problems soll nachfolgend - rein beispielhaft - anhand des Nitrierprozesses von Toluol oder Mononitrotoluolen (MNT) zu Dinitrotoluolen (DNT) veranschaulicht werden (vgl. H. Hermann et al., "Industrial Nitration of Toluene to Dinitrotoluene" in: ACS Symposium Series 623, "Nitration - Recent Laboratory and Industrial Development", Editors: L.F. Albright et al., Chapter 21, American Chemical Society, 1996, Washington D.C.), wobei die vorliegende Erfindung hierauf nicht beschränkt ist, sondern vielmehr universell auf Nitrierprozesse von nitrierbaren aromatischen Verbindungen im allgemeinen anwendbar ist, für welche die nachfolgenden Ausführungen entsprechend gelten.

Beispielsweise kann DNT aus Toluol in einem zweistufigen isothermen Verfahren im Gegenstrom hergestellt werden, wobei in den Nitriersäuren stets ein Überschuß an Salpetersäure von 1,01 bis 1,08 der benötigten Menge vorhanden sein muß, um einen quantitativen Umsatz der zu nitrierenden Stoffe (Toluol bzw. MNT) auf der jeweiligen Stufe zu erreichen. In der ersten Nitrierstufe (MNT-Stufe) wird bei Temperaturen zwischen 25 und 45 °C Toluol mit der DNT-Endsäure aus der DNT-Stufe und frischer Salpetersäure selektiv zu MNT umgesetzt. Die nach beendeter Reaktion in der ersten Nitrierstufe anfallende Nitriersäure (MNT-Endsäure) enthält im Gleichgewicht mit der organischen Phase noch 70 bis 73 % Schwefelsäure, ca. 0,3 bis 0,7 % Salpetersäure und 0,4 bis 2,0 % Nitrose als HNO₂. Nach Phasentrennung wird die MNT-Endsäure einer MNT-Endsäurebehandlung zugeführt, um noch die Restmenge an Salpetersäure, die Nitrose und das gelöste MNT/DNT zu entfernen. Das Roh-MNT, das noch Nitrokresole, Salpetersäure, Nitrose und andere Abbauprodukte enthält, wird direkt ohne Reinigung in der zweiten Stufe mit einer frischen Mischsäure aus 96 % Schwefelsäure und etwa 98 % bzw. 60 bis 68 % Salpetersäure zum DNT umgesetzt. Das nach Abtrennung der Nitriersäure ("DNT-Endsäure") anfallende Roh-DNT enthält neben dem DNT-Isomerengemisch aus etwa 76 % 2,4-DNT, 19,5 % 2,6-DNT, 0,6 % 2,5-DNT, 3,7 % "Ortho-Isomeren" (2,3-DNT und 3,4-DNT) und 0,8 % 3,5-DNT, maximal 0,01 % MNT und Spuren von TNT (z. B. 0,02 %) noch Nitrokresole, Salpetersäure, Schwefelsäure und gelöstes Stickstoffdioxid (NO₂) (vgl. z. B. US 4 482 769 A). Die Menge der im Roh-DNT gelösten und suspendierten Salpetersäure, Schwefelsäure und Nitrose hängt von der Zusammensetzung der DNT-Endsäure und der Qualität der Phasentrennung zwischen der DNT-Endsäure und dem Roh-DNT nach beendeter Umsetzung ab.

Um diese Stoffe zu entfernen, wird das nach der Nitrierung von Toluol zu DNT nach Abtrennung der Nitriersäure anfallende Roh-DNT, welches noch Schwefelsäure, Salpetersäure, Stickstoffoxide ("Nitrose"), Kresole sowie weitere Abbauprodukte enthält, durch Wäsche mit Wasser in mehreren Stufen von den vorgenannten Verunreinigungen befreit. Dies wird im Stand der Technik durch ein Standardverfahren, wie es vom grundlegenden Prinzip beispielsweise in A. B. Quakenbusch und B. T. Pennington, "The Olin Dinitrotoluene (DNT) Process", Polyurethanes World Congress, 10.-13. Oktober 1993, beschrieben ist, in einer dreistufigen Wäsche erreicht: Die Wäsche wird bei 60 bis 70 °C in drei Stufen durchgeführt, wobei in der ersten Stufe (saure Wäsche) mit Wasser alle Säuren, wie Schwefelsäure, Salpetersäure und Salpetriger Säure bzw. Nitrose, ausgewaschen werden, in der zweiten Waschstufe (Alkaliwäsche) mit Natriumcarbonat-Lösungen alle schwach sauren Stoffe, wie z. B. Nitrokresole, ausgewaschen werden und schließlich in der letzten Waschstufe mit Wasser die Spuren an Natriumcarbonat und Kresolen entfernt werden. Mit der Optimierung der ersten Stufe (saure Wäsche) befaßt sich die vorliegende Erfindung.

Die mit DNT gesättigten, vereinigten Abwässer aus der Wäsche des Roh-DNT sind - aufgrund des hohen Gehaltes an Säuren im Roh-DNT - stets sauer und enthalten je nach der Menge des Waschwassers (1,5 bis 3 m³/DNT) im Verhältnis zu dem gewaschenen DNT etwa 0,2 bis 3,0 % Schwefelsäure, 1 bis 3 % Salpetersäure, 0,1 bis 0,2 % HNO₂, 0,2 bis 0,5 % DNT (je nach Temperatur und Säuregehalt), 400 bis 800 ppm Nitrokresole und andere Abbauprodukte aus der Oxidation der Nitrokresole in der DNT-Stufe. Neben den toxischen Nitrokresolen, die vor Abgabe des Abwassers in einen Vorfluter entfernt werden müssen, stellt auch der hohe Gehalt an Salpetersäure von 1 bis 3 % und Schwefelsäure von 0,2 bis 3 % im Abwasser ein Problem dar. Sowohl die im Abwasser vorhandene Salpetersäuremenge als auch die in der Abfallsäure vorhandene Restsalpetersäuremenge und Nitrose sind für den Nitrierprozeß verloren und fallen in Abhängigkeit von der Behandlungsmethodik als mehr oder weniger verdünnte Salpetersäure an, die nicht unmittelbar in die Nitrierung zurückgeführt werden kann. So wird aus einer MNT-Endsäure durch Flush-Verdampfung im Vakuum eine dünne Salpetersäure mit ca. 16 % Salpetersäure erhalten oder durch Strippen bei Normaldruck ein ZweiphasenGemisch mit 15 bis 30 % Salpetersäure und MNT/DNT als organischer Phase.

Vor Abgabe dieser Abwässer aus der sauren Wäsche des DNT bzw. aus der Reinigung der MNT-Endsäure in einen Vorfluter müssen nicht nur das gelöste MNT/DNT, sondern auch der hohe Nitrat- und Sulfatgehalt auf die einzuhaltenden bzw. gesetzlich gültigen Grenzwerte für die Einleitung von Abwässern (z. B. 50 mg/m³ für Nitrat, 1200 mg für Sulfat und CSB 175 mg O₂/l), reduziert werden. Dies ist nur möglich durch eine Behandlung des Abwassers mit Calciumhydroxid zum Zwecke der Abtrennung des überschüssigen Sulfats als Calciumsulfat, mit nachfolgendem biologischem Abbau des Nitrates und der organischen Belastung.

Im Stand der Technik wurde vorgeschlagen, die Entfernung der sauren Komponenten aus dem Roh-DNT mittels Extraktionsverfahren durchzuführen. Dies ist jedoch nicht trivial. Denn bei einer Extraktion der sauren Komponenten aus dem Roh-DNT handelt es sich nicht um eine einfache Extraktion, bei der für jede Komponente über den gesamten Konzentrationsbereich ein Verteilungskoeffizient für die Verteilung zwischen den Phasen wirksam ist, sondern um ein sehr komplexes System. Alle Komponenten werden durch Umsetzung mit Wasser nach den Gleichungen (1) bis (3) über das Verteilungsgleichgewicht dieser Komponenten zwischen organischer Phase (Roh-DNT) einerseits und Waschwasser andererseits aus dem Roh-DNT entfernt:

(1) H₂SO₄ + H₂O ⇄ H₃O⁺ + HSO₄⁻

(2) HNO₃ + H₂O ⇄ H₃O⁺ + NO₃⁻

(3) NO₂ + H₂O → 2 HNO₃ + NO

Diese Gleichgewichte nach den Gleichungen (1) bis (3) beeinflussen sich gegenseitig, und zwar in Abhängigkeit von den Konzentrationen der einzelnen Komponenten. Zusätzlich hängen die Verteilungsgleichgewichte zwischen organischer Phase und wäßriger Phase von den Konzentrationen dieser Komponenten in der wäßrigen Phase ab. Bei niedrigen Konzentrationen liegen Schwefelsäure und Salpetersäure vollständig nach den obigen Gleichungen (1) und (2) dissoziiert im wäßrigen Extrakt vor; der Verteilungskoeffizient zwischen wäßriger Phase und organischer Phase ist hoch, und das Verteilungsgleichgewicht zwischen den beiden Phasen liegt vollständig auf der Seite des wäßrigen Extraktes. Die vollständige Extraktion von NO₂ hängt von der irreversiblen Disproportionierung von NO₂ nach Gleichung (3) ab; die Geschwindigkeit dieser Disproportionierung wird von der Säurestärke in der wäßrigen Phase beeinflußt und ist gleichermaßen stark von der Verweilzeit abhängig.

Aufgrund des betreffenden Verteilungsgleichgewichtes wird Schwefelsäure bis zu einer Konzentration von ca. 70 % im wäßrigen Extrakt fast vollständig aus der organischen Phase extrahiert; eine Rückextraktion findet quasi nicht statt. Erst ab ca. 70 % Schwefelsäure findet mit steigender Konzentration der Schwefelsäure eine Rückextraktion in die Organphase statt. Dies ist beispielsweise der Grund dafür, daß im Roh-DNT im Gleichgewicht mit einer DNT-Endsäure mit einem Schwefelsäuregehalt von über 81 % (Verhältnis Schwefelsäure/Wasser) schon bis zu 0,7 % Schwefelsäure im DNT gelöst vorliegen. Dagegen liegt Salpetersäure schon bei relativ niedrigen Konzentration als nichtdissoziierte Salpetersäure in wäßriger Lösung vor und wird dann als solche - entsprechend dem Verteilungsgleichgewicht - in die organische Phase rückextrahiert. Der Verteilungskoeffizient für Salpetersäure zwischen wäßriger Phase und organischer Phase wird mit zunehmender Konzentration der ausgewaschenen Säuren in der Wasserphase immer niedriger; dieser Effekt wird durch die Gegenwart von Schwefelsäure gravierend verstärkt, so daß der vorgenannte Verteilungskoeffizient für Salpetersäure zwischen wäßriger und organischer Phase in Gegenwart von Schwefelsäure sogar Werte kleiner als 1 annehmen kann. Im Gemisch von Schwefelsäure und Salpetersäure in Wasser wird nämlich das Dissoziationsgleichgewicht für Salpetersäure gemäß obiger Gleichung (2) schon bei niedrigeren Konzentrationen an Salpetersäure in der Wasserphase auf die Seite der nichtdissoziierten Salpetersäure verschoben, die bevorzugt in die organische Phase zurückextrahiert wird.

Das Stickstoffdioxid (NO₂) disproportioniert in der wäßrigen Phase quasi irreversibel nach obiger Gleichung (3) zu Salpetersäure. Aber auch diese Proportionierung nach obiger Gleichung (3) erfolgt nur quantitativ in Gegenwart von viel Wasser. In Gegenwart von höher konzentrierten Schwefelsäuren dagegen erfolgt die Disproportionierung nicht nach Gleichung (3) sondern nach den nachfolgenden Gleichungen (4) und (5):

(4) 2 NO₂ + H₂O → HNO₃ + HNO₂

(5) HNO₂ + H₂SO₄ → H₂O + NOHSO₄

Das HNO₂ in Gleichung (4) wird durch Bildung des Salzes NOHSO₄ nach obiger Gleichung (5) abgefangen, so daß eine weitere Disproportionierung zu HNO₃ und NO gemäß der nachfolgenden Gleichung (6):

(6) 3 HNO₂ → HNO₃ + 2 NO + H₂O

nicht mehr erfolgt bzw. allenfalls nur sehr langsam.

Dieses Verhalten ist insbesondere der Grund dafür, daß in allen Endsäuren aus der Nitrierung von 65 % Schwefelsäuregehalt an aufwärts neben Salpetersäure noch Nitrosylschwefelsäure (NOHSO₄) vorliegt, welche bei der Rekonzentrierung dieser Endsäuren z. B. durch aufwendiges Strippen mit Dampf oder mit anderen Methoden entfernt werden muß.

Diese komplexen Eigenschaften des Gemisches aus Salpetersäure, Schwefelsäure und Stickoxiden im Roh-DNT und deren gegenseitige Wechselwirkung bei der Extraktion erschweren eine Lösung des zuvor geschilderten Problems.

Um den Verlust an Salpetersäure von ca. 8 % der für die Nitrierung benötigten Menge und damit den Aufwand bei der Entfernung der Salpetersäure aus dem Abwasser zu reduzieren, wurde im Stand der Technik vorgeschlagen, die z. B. bei der Reinigung der MNT-Endsäure durch eine Flush-Verdampfung anfallende dünne Salpetersäure von ca. 16,3 % Salpetersäure und ca. 4,9 % HNO₂ durch Destillation auf eine 65%ige Salpetersäure aufzukonzentrieren, die wieder in die Nitrierung zurückgeführt werden kann (vgl. EP 0 415 354 A2).

Weiterhin wurde gemäß EP 0 279 312 A2 versucht, durch eine Wäsche des Roh-DNT mit bis zu 10 % Wasser die im Roh-DNT gelöste bzw. suspendierte Salpetersäure, Schwefelsäure und Nitrose so zurückzugewinnen, daß die bei dieser Wäsche anfallende konzentrierte Waschsäure mit einer Dichte über der des DNT unmittelbar in die Nitrierung zurückgeführt werden kann. Ziel der EP 0 279 312 A2 ist es somit, durch ein möglichst großes DNT/Waschwasser-Verhältnis auch in dem vereinigten Säureextrakt aus der zweistufigen Extraktion aus Roh-DNT möglichst hohe Säurekonzentrationen zu erzielen, so daß die zurückgeführte Säurephase ohne spezielle Aufkonzentrierung in der ersten Nitrierstufe als Mischsäure eingesetzt werden kann - was jedoch auf Kosten der nur unvollständigen Rückgewinnung von Salpetersäure geschieht: Durch diese Verfahrensweise können nämlich nur etwa 50 bis 72 % der im Roh-DNT gelösten Salpetersäure zurückgewonnen werden; die noch im DNT verbleibende Menge an Salpetersäure von 0,3 bis 0,6 % (3 - 6 kg/t DNT) gelangen weiterhin über die Wäsche ins Abwasser und stellen eine erhebliche Belastung dar. Weiterhin findet gemäß EP 0 279 312 A2 keine Rückgewinnung bzw. Berücksichtigung der Stickoxide statt; das Verfahren ist somit bezüglich seiner Verfahrensführung nicht darauf optimiert, die gleichermaßen enthaltenen Stickstoffoxide und die hieraus durch Disproportionierung erzeugte Salpetersäure in ausreichendem Maße zurückzugewinnen, was die relativ schlechten Ausbeuten in bezug auf die Salpetersäurerückgewinnung erklärt. Nach den Ausführungsbeispielen 1 und 2 der EP 0 279 312 A2 wird zwar die Sulfatfracht im Abwasser um ca. 96 bis 97 % erniedrigt, aber die Nitratfracht um nur ca. 60 bzw. 72 %: Es gelangen immer noch beträchtliche Mengen an Nitrat ins Abwasser (ca. 3,0 kg/t DNT in Beispiel 1 und 5,2 kg/t DNT in Beispiel 2), die neben zusätzlichen Behandlungsmaßnahmen für dieses Abwasser für die Nitratentfernung auch einen Verlust an Salpetersäure für die Nitrierung bedeutet. Somit nimmt das in der EP 0 279 312 A2 beschriebene Verfahren mit dem angeblichen Hauptziel einer möglichst quantitativen Rückgewinnung der Schwefelsäure aus dem Roh-DNT und der Erzielung eines Extrakts mit einer Säurekonzentration, die so hoch ist, daß bei Rückführung in die Nitrierung das Nitriergleichgewicht im Hinblick auf den Wassergehalt in der Nitriersäure möglichst wenig zu höheren Wassergehalten in der Nitriersäure verschoben wird, billigend in Kauf, daß die Rückgewinnung von Salpetersäure nicht quantitativ erfolgt, so daß eine Reduzierung der Nitratfracht im Abwasser auf ein Minimum zur Vermeidung zusätzlicher Behandlungsmaßnahmen zu Zwecken der Entfernung des Nitrats überhaupt nicht erreicht werden kann.

Zur Rückgewinnung der Salpetersäure aus dem Roh-DNT wurde ferner gemäß US 4 257 986 A versucht, durch Wäsche des Roh-DNT mit einer gereinigten MNT-Endsäure einen Teil der Salpetersäure aus dem Roh-DNT zu extrahieren und anschließend diese mit Salpetersäure beladene MNT-Endsäure wieder in die Nitrierung zurückzuführen. Mit diesem Verfahren können die im Roh-DNT gelöste Salpetersäure und Nitrose nicht vollständig, sondern nur entsprechend dem Verteilungsgleichgewicht für diese Stoffe zwischen dem DNT und der Säure extrahiert werden (ca. 50 bis 60 % der vorhandenen Salpetersäure).

Des weiteren wurde gemäß EP 0 155 586 A1 versucht, durch Änderung der Nitrierbedingungen die Verluste an Salpetersäure durch Extraktion mit dem Roh-DNT bzw. die Bildung von Nitrose aus Salpetersäure durch Oxidation der im Roh-MNT enthaltenen Dinitrokresole (vorwiegend 2,4-Dinitroparakresol) zu reduzieren, indem die Nitrierung des Toluols zum MNT nicht bei ca. 70 bis 72 % sondern bei 72 bis 76 % Schwefelsäure in der MNT-Endsäure durchgeführt wird. Durch diese Nitrierung des Toluols zu MNT in einer MNT-Endsäure mit einem Schwefelsäuregehalt von ca. 75 % werden bis zu 25 % weniger Dinitrokresol gebildet als bei der Nitrierung in einer MNT-Endsäure von 70 % Schwefelsäure (vgl. Hanson et al., ACS Symposium Series No. 22, 132 (1976), Industrial and Laboratory Nitration).

Alle vorgenannten Maßnahmen tragen dazu bei, die Verluste an Salpetersäure bei der Nitrierung von Toluol zu DNT durch Austrag aus dem Nitrierprozeß zu reduzieren. Aber sowohl die Wäsche des Roh-DNT nach der EP 0 279 312 A2 als auch die Aufkonzentrierung der aus der Flush-Verdampfung von MNT-Endsäure erhaltenen Salpetersäure nach der EP 0 415 354 A2 führen auf den jeweiligen Teilstufen lediglich zu einer Reduzierung des Salpetersäureverlustes. In bezug auf den Gesamtprozeß (Nitrierung, Endsäurebehandlung und Wäsche) werden immer noch Abwässer mit hohen Nitratkonzentrationen erhalten.

Die kanadische Patentschrift CA 1 034 603 A beschreibt ein Verfahren zur Herstellung von Toluylendiamin, bei dem Toluol in Gegenwart einer anorganischen Säure nitriert wird und danach die so gebildeten Rohdinitrotoluole mit Wasser, das frei von säureneutralisierenden Verbindungen sein soll, gewaschen werden, um die überschüssigen Säuren vor der katalytischen Reduzierung der Dinitrotoluole zu den Diaminen zu entfernen. Der gesamte resultierende wäßrige Extrakt fällt anschließend als Abwasser an. Eine Wiederverwendung und Rückführung des Extraktes kommt bei dem Verfahren nach der CA 1 034 603 A aus Rentabilitätsgründen nicht in Betracht, weil das gesamte Waschwasser mit so geringer Konzentration an Schwefelsäure, Salpetersäure, Salpetriger Säure und Nitroaromaten anfällt, daß ein Rezyklieren gänzlich ausscheidet: Die Waschlösung ist also derart verdünnt, daß ein Aufkonzentrieren nicht lohnt; deshalb wird sie als Abwasser verworfen. Zudem bleiben bei diesem Verfahren die Dinitrokresole im DNT, weil sie nicht durch eine Wäsche mit Alkali vor der Weiterverarbeitung zusätzlich entfernt werden. Die im DNT gelösten Nitrokresole werden erst als Sumpfprodukt bei der Destillation des letztlich herzustellenden Toluylendiamins (TDA) ohne zusätzlichen Aufwand mit anderen Rückständen aus dem Prozeß ausgeschleust. Die Abtrennung von Salpetersäure ohne Rückführung in den Nitrierprozeß wird lediglich als Teilschritt in der Herstellung von TDA beschrieben.

Bei allen bekannten Maßnahmen des Standes der Technik handelt es sich somit nur um Teillösungen, um den Verlust an Salpetersäure für die Nitrierung und damit die Belastung des Abwassers aus einer DNT-Anlage mit Nitrat zu vermeiden.

Schließlich beschreibt die auf die Anmelderin selbst zurückgehende EP 0 736 514 A2 ein Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen, wobei die rohen Dinitrotoluole mit einer verdünnten wäßrigen Lösung von Salpetersäure, Schwefelsäure und Salpetriger Säure, deren Dichte geringer ist als die der Dinitrotoluole, mehrstufig im Gegenstrom extrahiert werden, wobei das Volumenverhältnis der Dinitrotoluole zu der wäßrigen Lösung jeweils 1 : 3 bis 10 : 1 beträgt, und der wäßrige Extrakt direkt oder nach Aufkonzentrierung in die Nitrierung zurückgeführt wird.

Der Erfindung liegt daher die Aufgabe zugrunde, ein Verfahren zur Entfernung und Rückgewinnung von Nitriersäure, insbesondere Gemischen von Schwefelsäure, Salpetersäure und Stickoxiden, aus Nitrierprozessen und eine entsprechende Anlage zur Durchführung dieses Verfahrens bereitzustellen. Technischer Hintergrund des Streitpatents ist demnach das Entfernen und Wiedergewinnen von Schwefelsäure, Salpetersäure und Stickoxiden aus den bei der Nitrierung von nitrierbaren aromatischen Verbindungen nach Abtrennung der Nitriersäure anfallenden nitrierten Rohprodukten und die vorzugsweise direkte Rückführung in den Nitrierprozeß.

Insbesondere liegt eine Aufgabe der vorliegenden Erfindung darin, die Wäsche der durch Nitrierung nitrierbarer aromatischer Verbindungen erhaltenen nitrierten Rohprodukte zur Entfernung der darin gelösten Salpetersäure, Schwefelsäure und Stickoxide so durchzufiihren, daß diese Säuren einschließlich der Stickoxide nahezu vollständig wiedergewonnen werden können und das Abwasser nicht mehr belasten. Dabei sollte die rückgewonnene Nitriersäure vorteilhafterweise einen möglichst hohen Säuregehalt besitzen, um die rückgewonnenen Säuren einschließlich Stickoxiden nach Möglichkeit ohne Aufkonzentrierung in die Nitrierung zurückführen zu können, und besonders bevorzugt eine Zusammensetzung aufweisen, die einer Nitriersäure möglichst nahekommt, um die mit den rückgewonnenen Säuren in das Nitriergemisch zusätzlich eingetragene Wassermenge möglichst gering zu halten.

Eine weitere Aufgabe der vorliegenden Erfindung liegt in der Bereitstellung eines Verfahrens zur Entfernung und Rückgewinnung von Nitriersäure, insbesondere Gemischen von Schwefelsäure, Salpetersäure und Stickoxiden, aus den bei der Nitrierung von Toluol oder Mononitrotoluolen (MNT) nach Abtrennung der Nitriersäure anfallenden rohen Dinitrotoluole (DNT).

Schließlich liegt eine weitere Aufgabe der Erfindung darin, das in der auf die Anmelderin selbst zurückgehenden EP 0 736 514 A2 beschriebene Verfahren weiterzuentwickeln und zu optimieren, insbesondere im Hinblick auf eine vielfältigere Anwendungsbezogenheit für Nitrierprozesse im allgemeinen.

Die Anmelderin hat in überraschender Weise herausgefunden, daß sich die zuvor geschilderte Problemstellung dadurch lösen läßt, daß man die aus Nitrierprozessen stammenden, noch beträchtliche Mengen an Schwefelsäure, Salpetersäure und Stickoxiden bzw. Salpetriger Säure enthaltenden nitrierten Rohprodukte nach Abtrennung des Nitriersäuregemisches einem mehrstufigen Extraktionsverfahren unterzieht, welches eine Kombination von Querstromextraktion mit nachgeschalteter Gegenstromextraktion vorsieht.

Gegenstand der vorliegenden Erfindung ist - gemäß einem ersten Aspekt der vorliegenden Erfindung - ein Verfahren zum Entfernen und Wiedergewinnen von Nitriersäuregemischen, insbesondere Salpetersäure, Schwefelsäure und Stickoxiden, aus den bei der Nitrierung von nitrierbaren aromatischen Verbindungen nach Abtrennung der Nitriersäure anfallenden nitrierten Rohprodukten durch ein mehrstufiges Extraktionsverfahren gemäß Anspruch 1; weitere, vorteilhafte Ausgestaltungen des erfindungsgemäßen Verfahrens sind Gegenstand der Verfahrensunteransprüche. Weiterer Gegenstand der vorliegenden Erfindung - gemäß einem zweiten Aspekt der vorliegenden Erfindung - ist eine Produktionsanlage zur Nitrierung nitrierbarer aromatischer Verbindungen mit nachfolgender Aufreinigung der nitrierten Produkte einschließlich einer Entfernung und Wiedergewinnung von Nitriersäuregemischen, insbesondere Schwefelsäure, Salpetersäure und Stickoxiden, gemäß Anspruch 10.

Gegenstand der vorliegenden Erfindung - gemäß einem ersten Aspekt der vorliegenden Erfindung - ist somit ein Verfahren zum Entfernen und Wiedergewinnen von Nitriersäuregemischen, insbesondere Salpetersäure, Schwefelsäure und Stickoxiden bzw. Salpetriger Säure, aus den bei der Nitrierung von nitrierbaren aromatischen Verbindungen nach Abtrennung der Nitriersäure anfallenden nitrierten Rohprodukten durch ein mehrstufiges Extraktionsverfahren, wobei das Extraktionsverfahren mindestens zwei Stufen umfaßt, von denen eine Stufe eine Querstromextraktion und eine andere Stufe eine Gegenstromextraktion umfaßt. Wie in der Beschreibungseinleitung zu der vorliegenden Erfindung ausgeführt, werden im Rahmen der vorliegenden Erfindung unter dem Begriff der nitrierbaren aromatischen Verbindungen insbesondere Benzol, Toluol, Chlorbenzol, Dichlorbenzole, Mononitrobenzol, Mononitrotoluole, Mononitrochlorbenzol, Dinitrotoluole etc. verstanden.

Mit anderen Worten werden nach dem erfindungsgemäßen Verfahren die nitrierten Rohprodukte - nach vorangehender Abtrennung der Nitriersäurephase (d. h. der sauren wäßrigen Phase) - einer sauren Wäsche mittels mehrstufigem Extraktionsverfahren unterworfen, wobei das Extraktionsverfahren mindestens zweistufig ausgebildet ist, wovon eine Stufe eine Querstromextraktion und eine andere Stufe eine Gegenstromextraktion umfaßt.

Dabei wird im allgemeinen die Gegenstromextraktion im Anschluß an die Querstromextraktion durchgeführt, d. h. also die Querstromextraktion vor der Gegenstromextraktion durchgeführt. Gemäß einer bevorzugten Ausführungsform werden Gegenstromextraktion und/oder Querstromextraktion, vorzugsweise Gegenstromextraktion und Querstromextraktion, jeweils als Flüssig/Flüssig-Extraktion durchgeführt.

Das erfindungsgemäße Verfahren erlaubt es überraschenderweise, dem zuvor geschilderten Problem Rechnung zu tragen, d. h. berücksichtigt insbesondere die große Abhängigkeit des Verteilungskoeffizienten für Salpetersäure zwischen wäßriger und organischer Phase vom Schwefelsäuregehalt in der Waschsäure und die im Stand der Technik dadurch verursachte schlechte Rückgewinnungsrate in bezug auf Salpetersäure und Stickoxide im Fall hoher Säurekonzentrationen in der Waschsäure bzw. die im Stand der Technik unter Umständen noch notwendige Aufkonzentrierung der Waschsäure vor Rückführung in die Nitrierung.

Das gesamte Extraktionsverfahren zur weitgehend quantitativen Rückgewinnung von Schwefelsäure, Salpetersäure und Stickoxiden aus den nitrierten Rohprodukten mit minimalem Einsatz von Waschwasser, d. h. sowohl die Querstromextraktion als auch die Gegenstromextraktion, wird erfindungsgemäß mit einer sauren, im allgemeinen hochkonzentrierten Waschsäure durchgefiihrt. Diese umfaßt eine wäßrige Lösung von Salpetersäure, Schwefelsäure und Salpetriger Säure bzw. Stickoxiden. Diese Waschsäure wird vorzugsweise in situ gebildet, wenn die nitrierten Rohprodukte - nach vorangehender Abtrennung der aus der Nitrierung stammenden wäßrigen Nitriersäurephase - mit Wasser in Kontakt gebracht bzw. vermischt werden, weil die nitrierten Rohprodukte noch beträchtliche Mengen an Schwefelsäure, Salpetersäure und Salpetriger Säure bzw. Stickoxiden enthalten, welche dann teilweise in die wäßrige Phase übergehen, so daß in situ die saure Waschsäure gebildet wird. Durch die Menge an den nitrierten Rohprodukten zugesetztem Wasser und eine Kreisführung der Waschsäure läßt sich das gewünschte Volumenverhältnis von nitrierten Rohprodukten zu Waschsäure (d. h. zur wäßrigen Lösung von Salpetersäure, Schwefelsäure und Salpetriger Säure) und deren Zusammensetzung bzw. Konzentration gezielt einstellen bzw. steuern, wie nachfolgend noch beschrieben. Zu diesem Zweck werden vorteilhafterweise definierte Bereiche von Volumenverhältnissen Rohprodukte/Waschsäure bzw. Rohprodukte/(Frisch-)Wasser eingehalten, damit eine effiziente und vollständige Entfernung und Wiedergewinnung der Nitriersäuregemische, insbesondere von Salpetersäure, Schwefelsäure und Salpetriger Säure bzw. Stickoxiden, ermöglicht wird.

Im allgemeinen wird das erfindungsgemäße Verfahren derart durchgeführt, daß die Gegenstromextraktion im Anschluß an die Querstromextraktion durchgeführt wird, d. h. die Querstromextraktion vor der Gegenstromextraktion erfolgt. Mit anderen Worten wird das von Salpetersäure, Schwefelsäure und Stickoxiden zu befreiende nitrierte Rohproduktgemisch zunächst einer sauren Wäsche mittels Querstromextraktion unterworfen, welcher sich dann eine saure Wäsche mittels Gegenstromextraktion anschließt, wobei sowohl bei der Querstromextraktion als auch bei der Gegenstromextraktion als Waschsäure eine wäßrige Lösung von Salpetersäure, Schwefelsäure und Salpetriger Säure eingesetzt wird, jedoch mit unterschiedlichen Volumenverhältnissen von nitrierten Rohprodukten zu wäßriger Lösung, wie nachfolgend noch im einzelnen beschrieben.

Die Durchführung einer vorgeschalteten Querstromextraktion einerseits und einer hierzu nachgeschalteten Gegenstromextraktion andererseits ist dem Fachmann - jeweils als solche - bekannt, so daß diesbezüglich nicht auf weitere Einzelheiten eingegangen zu werden braucht. Im übrigen kann zu weiteren diesbezüglichen Einzelheiten beispielsweise verwiesen werden auf Kirk-Othmer, Encyclopedia of Chemical Technology, Third Edition, Volume 9, John Wiley & Sons, 1980, Seiten 672 bis 716, "Extraction liquid-liquid" und die dort referierte Literatur sowie auf Römpp Chemielexikon, 10. Auflage, Georg Thieme Verlag, Stuttgart/New York, Band 2, 1997, Seite 1268, Stichwort: "Extraktion" und die dort referierte Literatur.

Während bei dem erfindungsgemäßen Verfahren im Rahmen der Querstromextraktion überwiegend Schwefelsäure und nur ein Teil der Salpetersäure und der Salpetrigen Säure bzw. der Stickoxide abgetrennt wird, wird im nachfolgenden, zweiten Verfahrensschritt der Gegenstromextraktion dann die wenige restliche, nach der Querstromextraktion noch verbleibende Schwefelsäure und vor allem im wesentlichen sämtliche nach der Querstromextraktion noch verbleibende Salpetersäure einschließlich Salpetriger Säure bzw. Stickoxiden entfernt. Mit anderen Worten enthält der aus der Querstromextraktion hervorgehende wäßrige Extrakt überwiegend Schwefelsäure und nur relativ geringe Mengen an Salpetersäure und Salpetriger Säure bzw. Stickoxiden, während der aus der nachfolgenden Gegenstromextraktion hervorgehende Extrakt anteilig überwiegend Salpetersäure und Salpetrige Säure bzw. Stickoxide und nur geringe Mengen Schwefelsäure enthält. Der Grund hierfür ist, daß bei der Querstromextraktion mit relativ hohen Waschsäurekonzentrationen gearbeitet wird, d. h. die Menge an den rohen nitrierten Produkten zugesetztem Wasser relativ gering ist, was aufgrund des zuvor geschilderten Verteilungsgleichgewichts zwar dazu führt, daß mit der Waschsäure große Mengen an Schwefelsäure entfernt werden können, jedoch nur relativ geringe Mengen an Salpetersäure und Salpetriger Säure bzw. Stickoxiden: Denn die Salpetersäure liegt schon bei relativ niedrigen Konzentrationen als nichtdissoziierte Salpetersäure in wäßriger Lösung vor und wird entsprechend dem Verteilungsgleichgewicht in die organische Phase rückextrahiert, wobei dieser Effekt durch die Gegenwart von Schwefelsäure gravierend verstärkt wird; Salpetersäure und Salpetrige Säure bzw. Stickoxide können aber dann ohne weiteres im nachfolgenden Verfahrensschritt der Gegenstromextraktion entfernt werden.

Im allgemeinen wird im Rahmen der vorliegenden Erfindung die Querstromextraktion einstufig durchgeführt, wohingegen die Gegenstromextraktion üblicherweise mehrstufig, insbesondere mindestens zweistufig, vorzugsweise zwei- bis vierstufig, durchgeführt wird, da dies die beste Verfahrensökonomie und -effizienz ermöglicht.

Was die Querstromextraktion anbelangt, so wird diese - wie zuvor beschrieben - im allgemeinen mit einer sauren Waschsäure, insbesondere mit einer wäßrigen Lösung von vorwiegend Schwefelsäure und wenig Salpetersäure und Salpetriger Säure, durchgeführt. Dabei wird das Volumenverhältnis der nitrierten Rohprodukte zu der wäßrigen Lösung von Salpetersäure, Schwefelsäure und Salpetriger Säure (d. h. das Volumenverhältnis von nitrierten Rohprodukten zu Waschsäure) im allgemeinen auf 200 : 1 bis 1 : 10, insbesondere 120 : 1 bis 1 : 5, vorzugsweise 80 : 1 bis 1 : 5, eingestellt. Dennoch kann es einzelfallbedingt oder anwendungsbezogen erforderlich sein, von den vorgenannten Volumenverhältnissen Waschsäure/nitrierte Rohprodukte abzuweichen, was im Ermessen des Fachmanns liegt. Im allgemeinen wird die Querstromextraktion einstufig durchgeführt; jedoch ist es grundsätzlich möglich, die Querstromextraktion mehrstufig, insbesondere zwei- bis vierstufig, durchzuführen. Eine einstufige Ausgestaltung der Querstromextraktion wird erfindungsgemäß aber bevorzugt.

Die Einstellung der vorgenannten Volumenverhältnisse bei der Querstromextraktion wird durch Zugabe von Wasser in den entsprechenden Mengen zu den nitrierten Rohprodukten und zusätzlich durch eine Kreisfiihrung der in situ gebildeten Waschsäure erreicht. Da die nitrierten Rohprodukte noch beträchtliche Mengen an Schwefelsäure, Salpetersäure und Salpetriger Säure enthalten, bildet sich bei Zugabe von Wasser die saure Waschsäure in situ.

Zur Erreichung der gewünschten Konzentrationen von Schwefelsäure, Salpetersäure und Salpetriger Säure in der bei der Querstromextraktion gebildeten Waschsäure wird Wasser im allgemeinen in einer Menge bis zu 10 Gew.-%, insbesondere in einer Menge von 0,1 bis 10 Gew.-%, vorzugsweise 0,5 bis 6 Gew.-%, bezogen auf die nitrierten Rohprodukte, zu den nitrierten Rohprodukten zugesetzt. Die Menge des benötigten Wassers für die Querstromextraktion hängt maßgeblich von der Menge der in den nitrierten Rohprodukten gelösten Schwefelsäure und der zusätzlich noch suspendierten Nitriersäure ab und wird im allgemeinen derart gewählt, daß sich bei der Querstromextraktion eine 40- bis 80%ige Schwefelsäure bildet, in der - entsprechend dem Verteilungskoeffizienten für Salpetersäure und Nitrose - noch in geringen Mengen Salpetersäure bzw. Nitrose gelöst vorliegen, während die Hauptmenge der Salpetersäure einschließlich Nitrose in den nitrierten Rohprodukten nach der Querstromextraktion verbleibt. Als Wasser kann grundsätzlich Frischwasser verwendet werden; erfindungsgemäß bevorzugt ist es jedoch, wenn aus dem Prozeß selbst entstandenes Wasser bzw. Kondensat (z. B. Brüdenkondensat) eingesetzt wird, wie nachfolgend noch beschrieben; ein solches Kondensat wird beispielsweise aus einer Aufkonzentrierung der aus der Gegenstromextraktion resultierenden wäßrigen Extrakte oder aus der Aufkonzentrierung der Schwefelsäure aus der Nitrierung erhalten. Auch ist es möglich nur einen Teil des zugesetzten Wassers aus Frischwasser zu erhalten und den anderen Teil aus Kondensat.

Der aus der Querstromextraktion abgezogene, saure wäßrige Extrakt, d. h. das aus der Querstromextraktion abgezogene Säuregemisch aus Schwefelsäure, Salpetersäure und Salpetriger Säure, enthält im allgemeinen einen Gesamtsäuregehalt, von 40 bis 80 Gew.-%, insbesondere 45 bis 71 Gew.-%. Der Schwefelsäureanteil liegt hierbei bei 45 bis 100 %, insbesondere bei 60 bis 97 %, bezogen auf das Gesamtsäuregemisch.

Durch die Querstromextraktion werden im allgemeinen mindestens 80 %, insbesondere mindestens 95 %, vorzugsweise mindestens 98 %, der in den nitrierten Rohprodukten vorhandenen Schwefelsäure und/oder mindestens 0,1 %, insbesondere mindestens 1 %, vorzugsweise mindestens 2 %, der in den nitrierten Produkten vorhandenen Salpetersäure und Salpetrigen Säure einschließlich Stickoxiden abgetrennt. Der aus der Querstromextraktion resultierende saure Extrakt bzw. das hieraus abgezogene Säuregemisch enthält also überwiegend Schwefelsäure.

Der aus der Querstromextraktion stammende wäßrige Extrakt kann direkt (d. h. ohne Aufkonzentrierung) oder aber nach Aufkonzentrierung, vorzugsweise direkt, der Nitrierung wieder zugeführt werden. Eine Aufkonzentrierung kann beispielsweise zusammen mit einer aus der Nitrierung stammenden Nitrierendsäure zu einer Schwefelsäure z. B. von 87 % bis 96 % H₂SO₄ erfolgen, insbesondere in einer sogenannten SAC-Anlage (SAC = Sulphuric Acid Concentration).

Die mittels Querstromextraktion behandelten nitrierten Rohprodukte werden anschließend (d. h. nach Entfernung des aus Querstromextraktion resultierenden sauren wäßrigen Extrakts) einer sauren Wäsche mittels Gegenstromextraktion zugeführt. Die Gegenstromextraktion wird mit einer sauren Waschlösung, im allgemeinen mit einer wäßrigen Lösung von Salpetersäure, Schwefelsäure und Salpetriger Säure, durchgeführt. Eine solche saure Waschsäure bildet sich in situ durch Zusetzen der entsprechenden Wassermenge zu den aus der Querstromextraktion hervorgehenden nitrierten Rohprodukten, da diese noch beträchtliche Mengen an den vorgenannten Säuren, vorwiegend Salpetersäure und Stickoxide und daneben geringe Mengen Schwefelsäure, enthalten. Im allgemeinen wird bei der sauren Wäsche mittels Gegenstromextraktion das Volumenverhältnis der nitrierten Rohprodukte, die aus der Querstromextraktion stammen, zu der Waschsäure (d. h. der wäßrigen Lösung von Salpetersäure, Schwefelsäure und Salpetriger Säure) auf Werte von 200 : 1 bis 1 : 4, insbesondere 50 : 1 bis 1 : 2, vorzugsweise 4 : 1 bis 1 : 1, eingestellt.

Zur Erreichung bzw. Einstellung der vorgenannten Volumenverhältnisse bei der Gegenstromextraktion wird den zuvor mittels Querstromextraktion vorgereinigten nitrierten Rohprodukte Wasser (z. B. Frischwasser und/oder aus dem Prozeß stammendes Kondensat) in den entsprechenden Mengen zugesetzt. Die Menge an den nitrierten Rohprodukten für die Gegenstromextraktion zugesetztem Wasser beträgt im allgemeinen 1 bis 10 Gew.-%, insbesondere 2 bis 9 Gew.-%, vorzugsweise 3 bis 8 Gew.-%, bezogen auf die nitrierten Rohprodukte.

Der aus der Gegenstromextraktion stammende, saure wäßrige Extrakt kann direkt (d. h. ohne Aufkonzentrierung) oder aber nach Aufkonzentrierung der Nitrierung wieder zugeführt werden. Bevorzugt ist eine Ausführungsform, welche ohne Aufkonzentrierung auskommt. Für den Fall, daß die Aufkonzentrierung des aus der Gegenstromextraktion stammenden wäßrigen Extrakts erforderlich sein sollte, wird die Aufkonzentrierung im allgemeinen bis zu einem Gesamtsäuregehalt von mindestens 40 Gew.-%, insbesondere mindestens 50 Gew.-%, vorzugsweise mindestens 65 Gew.-%, berechnet als Salpetersäure, durchgeführt; das aus der Aufkonzentrierung stammende Wasser bzw. Kondensat kann dann in den Extraktionskreislauf zurückgeführt werden, beispielsweise in die Querstromextraktion und/oder in die Gegenstromextraktion.

Der aus der Gegenstromextraktion stammende Extrakt, d. h. das aus der Gegenstromextraktion abgezogene Säuregemisch aus Schwefelsäure, Salpetersäure und Salpetriger Säure, weist im allgemeinen einen Gesamtsäuregehalt, berechnet als Salpetersäure von 15 bis 40 Gew.-%, insbesondere 30 bis 35 Gew.-%, auf.

Durch die Gegenstromextraktion wird die nach der Querstromextraktion noch verbleibende geringe Menge an Schwefelsäure zumindest im wesentlichen vollständig abgetrennt; Entsprechendes trifft auch auf die Salpetersäure und die Salpetrige Säure bzw. die Stickoxide zu, welche nach der Querstromextraktion noch in beträchtlichen Mengen in den nitrierten Rohprodukten vorliegen, aber im Rahmen der Gegenstromextraktion zumindest im wesentlichen vollständig aus den nitrierten Rohprodukten entfernt werden.

Gemäß einer besonderen Ausführungsform der vorliegenden Erfindung kann die Gegenstromextraktion als solche wiederum mehrstufig, insbesondere mindestens zweistufig, vorzugsweise zwei- bis vierstufig, durchgeführt werden. Bei dieser besonderen Ausführungsform wird in allen Stufen der Gegenstromextraktion das Volumenverhältnis der nitrierten Rohprodukte, welche aus der Querstromextraktion hervorgehen, zu der wäßrigen Lösung von Salpetersäure, Schwefelsäure und Salpetriger Säure auf die vorgenannten Werte von 200 : 1 bis 1 : 4, insbesondere 50 : 1 bis 1 : 2, vorzugsweise 4 : 1 bis 1 : 1, eingestellt. Bei dieser Ausführungsform, gemäß der die Gegenstromextraktion als solche mehrstufig durchgeführt wird, wird die verdünnte wäßrige Lösung von Salpetersäure, Schwefelsäure und Salpetriger Säure, d. h. die saure Waschsäure, vorteilhafterweise, insbesondere aus verfahrensökonomischen Gründen, innerhalb jeder Gegenstromextraktionsstufe im Kreislauf geführt; dies ermöglicht insbesondere eine verbesserte Phasentrennung und eine verbesserte Scheideeffizienz. Bei der besonderen Ausführungsform der mehrstufigen Gegenstromextraktion kann neben Frischwasser insbesondere aus dem Gesamtprozeß anfallendes Kondensat, vorzugsweise das bei der gegebenenfalls durchgeführten Aufkonzentrierung anfallende Kondensat und/oder aus der Aufkonzentrierung von Schwefelsäure aus der Nitrierung anfallendes Brüdenkondensat, wieder dem Extraktionskreislauf der verdünnten sauren Lösung der letzten Gegenstromextraktionsstufe zugesetzt werden. Bei der besonderen Ausführungsform der mehrstufigen Gegenstromextraktion kann das Volumenverhältnis der nitrierten Rohprodukte zu der wäßrigen Lösung von Salpetersäure, Schwefelsäure und Salpetriger Säure durch Kreisführung und kann die Konzentration der Waschsäure über die Zugabe von Wasser in den Extraktionskreislauf der verdünnten Lösung der letzten Gegenstromextraktionsstufe eingestellt werden. Zur Aufrechterhaltung des Volumenverhältnisses der nitrierten Rohprodukte zu der wäßrigen Lösung von Salpetersäure, Schwefelsäure und Salpetriger Säure in den einzelnen Waschstufen (Gegenstromextraktionsstufen) kann nach der Phasentrennung nitrierte Rohprodukte/Waschsäure die abgetrennte Waschsäure in die jeweilige Waschstufe zurückgeführt werden und nur der Überschuß in die nächste Waschstufe eingespeist werden.

Unabhängig davon, ob die Gegenstromextraktion einstufig oder aber mehrstufig durchgeführt wird, wird die Dichte der wäßrigen Lösung von Salpetersäure, Schwefelsäure und Salpetriger Säure jeweils geringer eingestellt als die Dichte der nitrierten Produkte. Dies ermöglicht eine zuverlässige Verfahrensführung. Im Unterschied hierzu kann es, je nach Säurekonzentration, im Rahmen der vorgeschalteten Querstromextraktion vorkommen, daß die Dichte der wäßrigen Lösung von Salpetersäure, Schwefelsäure und Salpetriger Säure größer als die Dichte der nitrierten Produkte ist, insbesondere bei sehr hohen Gesamtsäurekonzentrationen. Im Rahmen der nachfolgenden Gegenstromextraktion sollte dies aber aus den vorgenannten Gründen vermieden werden.

Die aus der Gegenstromextraktion resultierenden nitrierten Rohprodukte werden anschließend einer weiteren Aufreinigung bzw. Aufbereitung unterzogen. Diese erfolgt insbesondere mittels alkalischer und nachfolgender neutraler Wäsche, welcher sich wiederum gegebenenfalls einer Trocknung der nitrierten Produkte anschließen kann.

Es ist vollkommend überraschend, daß sich nach dem erfindungsgemäßen Verfahren Schwefelsäure, Salpetersäure und Stickoxide in mehr als 98%iger, vorzugsweise mehr als 99%iger Ausbeute aus den nitrierten Rohprodukten extrahieren lassen. Aufgrund der Tatsache, daß im vorgeschalteten Querextraktionsverfahrensschritt der überwiegende Anteil an Schwefelsäure entfernt wird, wird im zweiten, nachgeschalteten Verfahrensschritt der Gegenstromextraktion das Verteilungsgleichgewicht der Salpetersäure und der Salpetrigen Säure zugunsten der wäßrigen Phase verschoben, so daß sich - neben den Resten an Schwefelsäure - im Verfahrensschritt der Gegenstromextraktion Salpetersäure und Salpetrige Säure bzw. Stickoxide nahezu quantitativ entfernen lassen. Dies gelingt nur aufgrund der erfindungsgemäßen Kombination von saurer Wäsche mittels Querstromextraktion mit nachgeschalteter sauer Wäsche mittels Gegenstromextraktion in der zuvor beschriebenen Art und Weise.

Vorteilhafterweise wird in allen Extraktionsstufen, d. h. sowohl bei der Querstromextraktion als auch bei der Gegenstromextraktion, bei Temperaturen oberhalb der Schmelztemperaturen der nitrierten Produkte gearbeitet. Auf diese Weise können im Rahmen der Flüssig/Flüssig-Extraktion die verbleibenden Säureanteile zuverlässig aus den nitrierten Rohprodukten entfernt werden.

Für den Fall, daß im Rahmen der Querstromextraktion und/oder der Gegenstromextraktion eine Aufkonzentrierung der anfallenden, sauren wäßrigen Extrakte vorgesehen ist, ist es vorteilhaft, die anfallenden aufzukonzentrierenden Lösungen von Salpetersäure, Schwefelsäure und Salpetriger Säure aus allen Extraktionsstufen zu vereinen und gemeinsam aufzukonzentrieren, um sie nachfolgend der Nitrierung wieder zuzuführen. Zusammen mit den Lösungen aus allen Extraktionsstufen können auch die salpetersäurehaltigen Strippkondensate aus der Reinigung der Nitrierendsäure gemeinsam aufkonzentriert und nachfolgend der Nitrierung wieder zugeführt werden. Die Aufkonzentrierung erfolgt im allgemeinen bis zu einem Gesamtsäuregehalt von mindestens 40 Gew.-%, insbesondere mindestens 50 Gew.-%, vorzugsweise mindestens 65 Gew.-%, berechnet als Salpetersäure.

Das erfindungsgemäße Verfahren eignet sich insbesondere zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen, ist aber keinesfalls auf diese Ausführungsform beschränkt.

Das erfindungsgemäße Verfahren ermöglicht die effiziente Entfernung und Rückgewinnung von Nitriersäuregemischen, insbesondere Gemischen von Schwefelsäure, Salpetersäure und Stickoxiden, aus Nitrierprozessen, d. h. aus den nitrierten Rohprodukten, mit über 98%iger, vorzugsweise über 99%iger Ausbeute. Die vorgenannten Säuren einschließlich der Stickoxide können nahezu vollständig wiedergewonnen werden. Auf diese Weise wird das resultierende Abwasser nicht mehr belastet. Vielmehr können die rückgewonnenen Säuregemische wieder in die Nitrierung zurückgeführt werden. Dies wird erfindungsgemäß insbesondere dadurch ermöglicht, daß die rückgewonnen Säuregemische mit relativ hohen Konzentrationen anfallen, so daß ihre Rezyklierung ohne weiteres möglich ist. Durch die Kombination von Querstromextraktion mit nachgeschalteter Gegenstromextraktion wird die eingetragene Wassermenge möglichst gering gehalten, so daß ein relativ konzentriertes Nitriersäuregemisch entsteht, welches gegebenenfalls direkt wieder in die Nitrierung zurückgeführt werden kann.

Weiterer Gegenstand der vorliegenden Erfindung ist eine Produktionsanlage zur Nitrierung nitrierbarer aromatischer Verbindungen mit nachfolgender Aufreinigung der nitrierten Produkte einschließlich einer Entfernung und Wiedergewinnung von Nitriersäuregemischen, insbesondere Salpetersäure, Schwefelsäure und Stickoxiden, wobei die Produktionsanlage die folgenden Einheiten umfaßt:
a) eine Nitriereinheit (N) zur Nitrierung nitrierbarer aromatischer Verbindungen mit einem oder mehreren entsprechenden Reaktionsbehältern zur Durchführung der Nitrierreaktion(en); und,
b) in Produktionslinie stromabwärts zur Nitriereinheit (N) angeordnet, eine Einheit (WS) zur Durchführung einer sauren Wäsche mittels Extraktion, insbesondere zur Durchführung des zuvor beschriebenen Verfahrens, wobei die Einheit (WS)
   - eine Querstromextraktionseinheit (WS I) zur Durchführung einer sauren Wäsche von nitrierten Rohprodukten mittels Querstromextraktion sowie,
   - in Produktionslinie stromabwärts zur Querstromextraktionseinheit (WS I), eine Gegenstromextraktionseinheit (WS II) zur Durchführung einer sauren Wäsche der aus der Querstromextraktionseinheit (WS) hervorgehenden nitrierten Rohprodukte mittels Querstromextraktion
aufweist.

Zwischen der Nitriereinheit (N) und der Extraktionseinheit (WS) ist im allgemeinen noch eine Trenneinheit (S) zur Abtrennung der sauren wäßrigen Phase von der nitrierten Rohproduktphase vor Eintritt in die Extraktionseinheit (WS) vorgesehen, bevor die nitrierten Rohprodukte dann der Extraktionseinheit (WS) zugeführt werden.

Stromabwärts zur Extraktionseinheit (WS) für die saure Wäsche ist im allgemeinen noch eine Einheit (W) zur weitergehenden Aufbereitung und/oder Aufreinigung der aus der Extraktionseinheit (WS) hervorgehenden nitrierten Rohprodukte vorgesehen, welche insbesondere eine Einheit (WA) zur Durchführung einer alkalischen Wäsche mit einer nachgeschalteten Einheit (WN) zur Durchführung einer neutralen Wäsche und gegebenenfalls eine abschließende Einheit (T) zur Trocknung der gereinigten Endprodukte aufweist.

In bezug auf die erfindungsgemäße Produktionsanlage gelten die obigen Ausführungen zu dem erfindungsgemäßen Verfahren entsprechend, so daß zur Vermeidung von Wiederholungen hierauf Bezug genommen werden kann.

Weitere Vorteile, Eigenschaften, Aspekte und Merkmale der vorliegenden Erfindung ergeben sich aus der folgenden Beschreibung eines in den Zeichnungen dargestellten, bevorzugten Ausführungsbeispiels. Es zeigt:
- Fig. 1: eine schematische Darstellung eines Ablaufs des erfindungsgemäßen Verfahrens gemäß einem bevorzugten Ausführungsbeispiel der Erfindung;
- Fig. 2: eine schematische Darstellung einer erfindungsgemäßen Produktionsanlage zur Nitrierung nitrierbarer aromatischer Verbindungen mit nachfolgender Aufreinigung der nitrierten Produkte einschließlich einer Entfernung und Wiedergewinnung von Nitriersäuregemischen gemäß einem bevorzugten Ausführungsbeispiel der Erfindung.

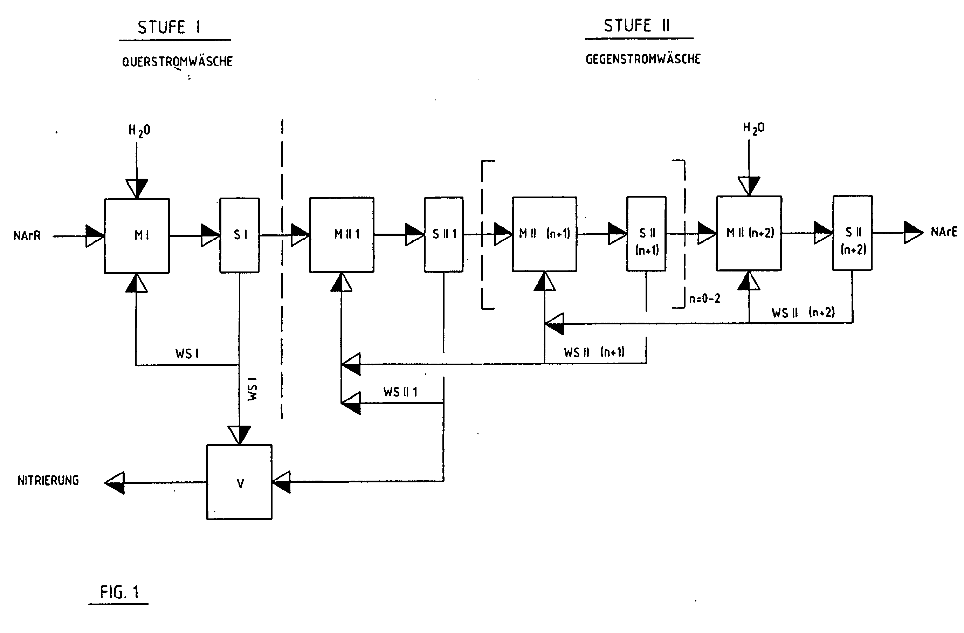

Fig. 1 zeigt ein Beispiel für das erfindungsgemäße Verfahren der zweistufigen Wäsche zur Rückgewinnung von Schwefelsäure, Salpetersäure und Nitrose aus Nitroaromaten. Der rohe Nitroaromat (NArR) aus der Nitrierung wird zusammen mit Frischwasser oder Kondensat aus einer Säureaufkonzentrierung und der im Kreis geführten Waschsäure (WS I) aus dem Scheider (S I) in den Mischer (M I) der Waschstufe I (Querstromwäsche) eingespeist. Im Separator (S I) wird die Emulsion aus Nitroaromat (NArR) und Waschsäure (WS I) getrennt. Die Waschsäure (WS I) wird in den Mischer (M I) so zurückgeführt, daß sich das vorgegebene Volumenverhältnis Waschsäure : Nitroaromat im Mischer (M I) einstellt. Der Überschuß an Waschsäure (WS I) wird in der Vorlage (V) gesammelt. Der Nitroaromat (NArR) aus dem Scheider (S I) wird in den Mischer (M II 1) der Waschstufe II (Gegenstromextraktion) zusammen mit der überschüssigen Waschsäure (WS II (n+1)) zusammen mit der im Kreis geführten Waschsäure (WS II 1) aus Scheider (S II 1) eingespeist. Nach Phasentrennung der Waschemulsion aus dem Mischer (M II 1) im Scheider (S II 1) wird die Waschsäure (WS II 1) in dem Mischer (M II 1) so zurückgeführt, daß sich das vorgegebene Volumenverhältnis Nitroaromat : Wasser im Mischer (M II 1) einstellt. Der Überschuß an Waschsäure (W II 1) wird in der Vorlage (V) gesammelt und zusammen mit der Waschsäure (WS I) direkt oder nach einer zusätzlichen Zwischenkonzentrierung in die Nitrierung zurückgeführt. Der Nitroaromat (NArR) aus dem Scheider (S II 1) wird in den Mischer (M II (n+1)) zusammen mit der im Kreis geführten Waschsäure (WS II (n+1)) aus dem Scheider (S II (n+1)) und dem Überschuß an Waschsäure aus dem Scheider (S II (n+2)) eingespeist. Nach Phasentrennung der Waschemulsion aus Mischer (M II (n+1)) in Scheider (n II+1) wird die Waschsäure (WS II (n+1)) so zurückgeführt, daß sich das vorgegebene Volumenverhältnis Nitroaromat : Waschsäure im Mischer (M II (n+1)) einstellt. Der Überschuß an Waschsäure (WS II (n+1)) wird in den Mischer (M II 1) eingespeist. Je nach Anzahl der Waschstufen wird dieser Prozeß n-mal wiederholt. Im letzten Mischer (M II (n+2)) wird der zu waschenden Nitroaromat aus dem Scheider (S II (n+2)) zusammen mit Frischwasser oder Kondensat aus einer Säureaufkonzentrierung und der Waschsäure (WS II (n+2)) aus Scheider (S II (n+2)) eingespeist. Nach Phasentrennung der Waschemulsion aus Mischer (M II (n+2)) in Scheider (S II (n+2)) wird die Waschsäure (WS II (n+2)) so zurückgeführt, daß sich das vorgegebene Volumenverhältnis Nitroaromat : Waschsäure im Mischer (M II (n+2)) einstellt. Der Überschuß an Waschsäure (WS II (n+2)) wird in den Mischer (M II (n+1)) eingespeist. Der von Säure befreite Nitroaromat (NArE) wird in die nachfolgende Behandlung mit Alkali abgegeben.

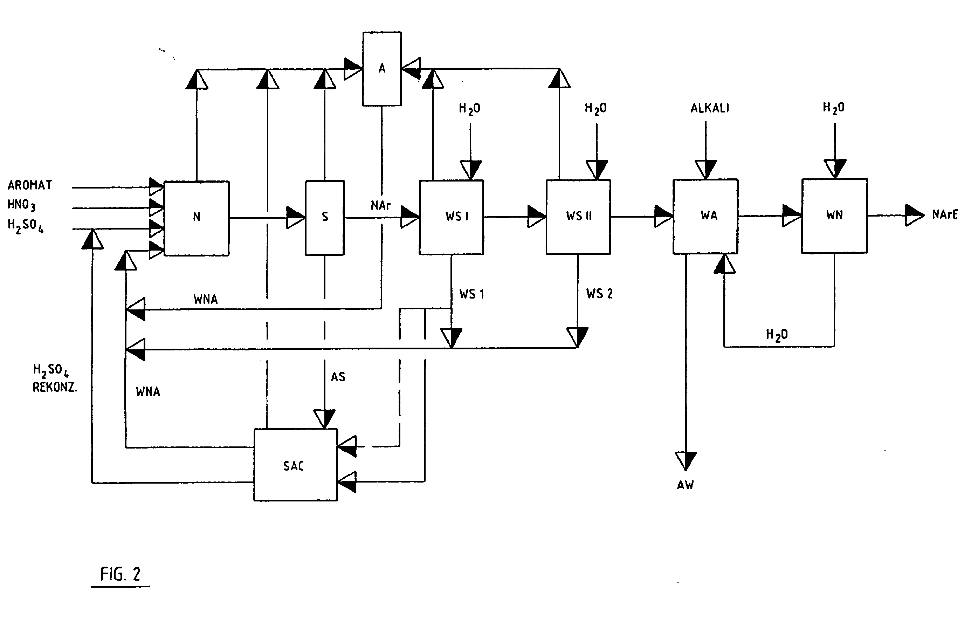

Fig. 2 zeigt ein Beispiel für eine Anlage mit integrierter erfindungsgemäße zweistufiger Rückgewinnung von Schwefelsäure, Salpetersäure und Nitrose aus dem rohen Nitroaromat. Der in der Nitriereinheit (N) durch die Umsetzung des Aromaten mit einem Gemisch aus Salpetersäure und Schwefelsäure gebildete Nitroaromat (NAr) wird nach Trennung der Nitrieremulsion im Scheider (S) in die Stufe I (Querstromwäsche) (WS I) der sauren Wäsche zusammen mit Wasser und/oder Kondensat aus einer Säureaufkonzentrierung eingespeist. Nach Phasentrennung der Waschemulsion in (WS I) wird der von Schwefelsäure befreite Nitroaromat in die Stufe II (Gegenstrom) (WS II) der sauren Wäsche eingespeist zusammen mit der für die Wäsche vorgesehenen Wassermenge und/oder Kondensat aus einer Säureaufkonzentrierung. Nach Phasentrennung der Waschemulsion in (WS II) wird der von Schwefelsäure, Salpetersäure und Nitrose befreite Nitroaromat in eine Wäsche mit Alkali (WA) und anschließend in eine Wäsche mit reinem Wasser (WN) gewaschen. Nach Phasentrennung in der Neutralwäsche (WN) wird der von Säuren, Phenolen und sonstigen Verunreinigungen befreite Nitroaromat (NArE) in die Weiterverarbeitung (vorwiegend einer Reduktion zu den entsprechenden Aminen) abgegeben. Das aus der Neutralwäsche (WN) abgegebene Waschwasser wird zusammen mit Alkali (z. B. Natronlauge) in die alkalische Wäsche (WA) eingespeist. Das in der Wäsche mit Alkali abgetrennte phenolhaltige Waschwasser (AW) wird einer Behandlung zugeführt. Die aus der erfindungsgemäßen sauren Wäsche in WS I und WS II abgegebenen Waschsäuren WS 1 und WS 2 werden getrennt oder gemeinsam zusammen mit der schwachen Salpetersäure (WNA) aus der Schwefelsäurerekonzentrierung (SAC) direkt oder nach Zwischenkonzentrierung und zusammen mit der Salpetersäure aus der Abgasreinigung (A) in die Nitrierung zurückgeführt. Die Waschsäure aus der ersten Waschstufe (WS I) kann alternativ auch zusammen mit der Nitrierendsäure in der SAC-Anlage zu einer Schwefelsäure mit 87 bis 96 % aufbereitet werden. Die aufkonzentrierte Schwefelsäure aus der SAC-Anlage wird gleichfalls in die Nitrierung zurückgeführt.

Weitere Ausgestaltungen, Abwandlungen, Variationen und Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand des folgenden Ausführungsbeispiels veranschaulicht, welches die vorliegende Erfindung jedoch keinesfalls beschränkt.

### Ausführungsbeispiel:

### Stufe I - Querstromextraktion

3.400 kg Roh-DNT mit einem Restgehalt von 1,85 % Schwefelsäure, 1,5 % Salpetersäure und 0,85 % Stickstoffdioxid nach Abtrennung der DNT-Endsäure werden mit einer Waschsäure der Zusammensetzung Schwefelsäure 55,6 %, Salpetersäure 3,07 % und Nitrose 3,52 % (als HNO₂) im Volumenverhältnis DNT : Waschsäure 1 : 2 einstufig gewaschen.

Gleichzeitig werden kontinuierlich ca. 42 kg Wasser/h zugesetzt, so daß sich die Konzentration der Waschsäure durch die aus dem Roh-DNT extrahierten Säure nicht ändert.

98 % der im Roh-DNT nach Abtrennung der DNT-Endsäure gelösten und emulgierten Schwefelsäure, ca. 6,7 % der Salpetersäure und ca. 13,5 % der Nitrose (als HNO₂) werden in dieser Waschstufe aus dem Roh-DNT entfernt.

Nach Phasentrennung der Waschemulsion wird die Waschsäure in die Wäsche zurückgeführt und die überschüssige Waschsäure in einer Vorlage gesammelt und von dort direkt oder zusammen mit der Säure aus Waschstufe II (Gegenstromextraktion) in die Nitrierung zurückgeführt oder mit einer aus der Nitrierung stammenden Nitrierendsäure vereint und nachfolgend in einer SAC-Anlage aufbereitet.

Das abgetrennte Roh-DNT (ca. 3.335 kg DNT/h) mit einem Gehalt von ca. 1,43 % Salpetersäure, 0,7 % Stickstoffdioxid und 0,04 % Schwefelsäure werden in der nachfolgenden Stufe II (Gegenstromextraktion) weiterbehandelt.

### Stufe II - Gegenstromextraktion

Etwa 3.335 kg Roh-DNT aus der Stufe I (Querstromextraktion) mit einem Gehalt an 0,37 kg Schwefelsäure, ca. 14 kg Salpetersäure und ca. 7,35 kg Stickstoffdioxid pro Tonne Roh-DNT werden zweistufig im Gegenstrom gewaschen.

In der ersten Waschstufe der insgesamt zweistufig durchgeführten Gegenstromextraktion wird das Roh-DNT mit einer Waschsäure der Zusammensetzung 34,22 % Salpetersäure, 0,63 % Schwefelsäure und 0,43 % Nitrose (als HNO₂) im Volumenverhältnis 1 : 1 gewaschen. Gleichzeitig wird aus der zweiten Wasch- bzw. Extraktionsstufe der Gegenstromextraktion soviel Waschsäure mit geringerer Säurekonzentration zugeführt, daß sich die Konzentration in der ersten Waschstufe nicht ändert.

In der zweiten Waschstufe der Gegenstromextraktion wird gleichfalls im Phasenverhältnis 1 : 1 mit einer Waschsäure mit der Konzentration gewaschen, mit der diese Waschsäure aus dieser Waschstufe in die ersten Waschstufe der Gegenstromextraktion abgegeben wird.

Die Menge des zugesetzten Wassers (im vorliegenden Beispiel ca. 1281 Wasser), das auf der letzten Extraktionsstufe der Gegenstromwäsche eingespeist wird, wird so gewählt, daß die Konzentration der Waschsäure in der ersten Extraktionsstufe in Kontakt mit dem zu waschenden DNT, die für diese Waschsäure festgelegte Säurestärke bzw. die festgelegte Dichte nicht überschreitet.

Um das gewünschte Volumenverhältnis in jeder Waschstufe von 1 : 2 (Stufe I - Querstromextraktion) bzw. 1 : 1 (Stufe II - Gegenstromextraktion) aufrechtzuerhalten, wird die aus dem Scheideteil z. B. eines Mixer-Settlers nach Trennung der Phasen ablaufende Waschsäure zurück in den Waschteil als Waschsäure eingespeist.

Die überschüssige Waschsäure aus der Extraktionsstufe der Gegenstromextraktion wird in der gleichen Vorlage wie die Waschsäure aus der Querstromextraktion gesammelt. Die vereinigten Säureextrakte mit einer Gesamtsäure von ca. 50 % (als Salpetersäure berechnet) werden direkt in die Nitrierung zurückgeführt oder können vor Rückführung in die Nitrierung zusätzlich in einer Destillationseinrichtung bis zu einer Säurekonzentration von maximal 65 % Gesamtsäure (als Salpetersäure berechnet) aufkonzentriert werden. Alternativ kann die überschüssige Waschsäure aus der ersten Waschstufe der Gegenstromextraktion aber auch separat aufgefangen und direkt in die Nitrierung zurückgeführt oder zusammen mit der verdünnten Salpetersäure aus der Reinigung der Nitrierendsäure in einer Destillationseinrichtung bis zu einer Säurekonzentration von maximal 65 % Gesamtsäure (als Salpetersäure berechnet) aufkonzentriert werden.

Das Brüdenkondensat mit ca. 0,3 bis 1 % an Säure (vor allem Salpetersäure) wird als Waschsäure in die zweite bzw. letzte Waschstufe der Gegenstromextraktion zurückgeführt.

Aus dem gewaschenen DNT werden neben der Schwefelsäure alle stickstoffhaltigen Säuren (HNO₃ und HNO₂) zu mehr als 98 % ausgewaschen und stehen damit für die Rückführung in die Nitrierung zur Verfügung.

## Patentansprüche

1. Verfahren zum Entfernen und Wiedergewinnen von Nitriersäuregemischen, insbesondere Salpetersäure, Schwefelsäure und Stickoxiden, aus den bei der Nitrierung von nitrierbaren aromatischen Verbindungen nach Abtrennung der Nitriersäure anfallenden nitrierten Rohprodukten durch ein mehrstufiges Extraktionsverfahren,
**dadurch gekennzeichnet,**
**daß** das Extraktionsverfahren mindestens zwei Stufen umfaßt, von denen eine Stufe eine Querstromextraktion und eine andere Stufe eine Gegenstromextraktion umfaßt.

2. Verfahren nach Anspruch 1, **dadurch gekennzeichnet, daß** die Gegenstromextraktion im Anschluß an die Querstromextraktion durchgeführt und/oder daß die Querstromextraktion und/oder die Gegenstromextraktion als Flüssig/Flüssig-Extraktion durchgeführt werden und/oder daß die Querstromextraktion einstufig und die Gegenstromextraktion mehrstufig durchgeführt wird.

3. Verfahren nach Anspruch 2, **dadurch gekennzeichnet, daß** die Querstromextraktion einstufig und die Gegenstromextraktion zwei- bis vierstufig durchgeführt wird und/oder daß die Querstromextraktion mit einer wäßrigen Lösung von Salpetersäure, Schwefelsäure und Salpetriger Säure durchgeführt wird, wobei das Volumenverhältnis der nitrierten Rohprodukte zu der wäßrigen Lösung von Salpetersäure, Schwefelsäure und Salpetriger Säure auf 200 : 1 bis 1 : 10 eingestellt wird.

4. Verfahren nach einem der Ansprüche 1 bis 3, **dadurch gekennzeichnet, daß** der aus der Querstromextraktion stammende wäßrige Extrakt direkt oder nach Aufkonzentrierung der Nitrierung wieder zugeführt wird und/oder daß das aus der Querstromextraktion abgezogene Säuregemisch aus Schwefelsäure, Salpetersäure und Salpetriger Säure einen Gesamtsäuregehalt von 40 bis 80 Gew.-% aufweist.

5. Verfahren nach Anspruch 4, **dadurch gekennzeichnet, daß** der aus der Querstromextraktion stammende wäßrige Extrakt direkt der Nitrierung wieder zugeführt wird und/oder daß das aus der Querstromextraktion abgezogene Säuregemisch aus Schwefelsäure, Salpetersäure und Salpetriger Säure einen Gesamtsäuregehalt von 45 bis 71 Gew.-% aufweist.

6. Verfahren nach einem der Ansprüche 1 bis 5, **dadurch gekennzeichnet, daß** die Gegenstromextraktion mit einer wäßrigen Lösung von Salpetersäure, Schwefelsäure und Salpetriger Säure durchgeführt wird, wobei das Volumenverhältnis der nitrierten Rohprodukte aus der Querstromextraktion zu der wäßrigen Lösung von Salpetersäure, Schwefelsäure und Salpetriger Säure auf 200 : 1 bis 1 : 4 eingestellt wird, und/oder daß die Einstellung des Volumenverhältnisses bei der Gegenstromextraktion durch Zugabe von Wasser in entsprechenden Mengen zu den nitrierten Rohprodukten und/oder durch Kreisführung der wäßrigen Lösung von Salpetersäure, Schwefelsäure und Salpetriger Säure erfolgt

7. Verfahren nach Anspruch 6, **dadurch gekennzeichnet, daß** das Volumenverhältnis der nitrierten Rohprodukte aus der Querstromextraktion zu der wäßrigen Lösung von Salpetersäure, Schwefelsäure und Salpetriger Säure auf 50 : 1 bis 1 : 2 eingestellt wird und/oder daß die Einstellung des Volumenverhältnisses bei der Gegenstromextraktion durch Zugabe von Wasser in Mengen von 1 bis 10 Gew.-%, bezogen auf die nitrierten Rohprodukte, erfolg.

8. Verfahren nach einem der Ansprüche 1 bis 7, **dadurch gekennzeichnet, daß** das aus der Gegenstromextraktion abgezogene Säuregemisch aus Schwefelsäure, Salpetersäure und Salpetriger Säure einen Gesamtsäuregehalt, berechnet als Salpetersäure, von 15 bis 40 Gew.-% aufweist und/oder daß die Gegenstromextraktion als solche mehrstufig durchgeführt wird.

9. Verfahren nach Anspruch 8, **dadurch gekennzeichnet, daß** das aus der Gegenstromextraktion abgezogene Säuregemisch aus Schwefelsäure, Salpetersäure und Salpetriger Säure einen Gesamtsäuregehalt, berechnet als Salpetersäure, von 30 bis 35 Gew.-%, aufweist und/oder daß in allen Stufen der Gegenstromextraktion das Volumenverhältnis der nitrierten Rohprodukte zu der wäßrigen Lösung von Salpetersäure, Schwefelsäure und Salpetriger Säure auf 200 : 1 bis 1 : 4 eingestellt wird.

10. Verfahren nach einem der Ansprüche 1 bis 9, **dadurch gekennzeichnet, daß** die Gegenstromextraktion derart geführt wird, daß die Dichte der wäßrigen Lösung von Salpetersäure, Schwefelsäure und Salpetriger Säure jeweils geringer eingestellt wird als die Dichte der nitrierten Produkte.

11. Verfahren nach einem der Ansprüche 1 bis 10, **dadurch gekennzeichnet, daß** der aus der Gegenstromextraktion stammende wäßrige Extrakt direkt oder nach Aufkonzentrierung der Nitrierung wieder zugeführt wird und/oder daß die mittels Gegenstromextraktion behandelten nitrierten Rohprodukte anschließend einer weiteren Aufreinigung und/oder Aufbereitung zugeführt werden.

12. Verfahren nach Anspruch 11, **dadurch gekennzeichnet, daß** im Falle der Aufkonzentrierung diese bis zu einem Gesamtsäuregehalt von mindestens 40 Gew.-%, berechnet als Salpetersäure, durchgeführt wird und/oder daß die weitere Aufreinigung und/oder Aufbereitung der mittels Gegenstromextraktion behandelten nitrierten Rohprodukte mittels alkalischer und nachfolgender neutraler Wäsche, gegebenenfalls mit anschließende Trocknung, erfolgt.

13. Verfahren nach einem der Ansprüche 1 bis 12, **dadurch gekennzeichnet, daß** in allen Extraktionsstufen bei einer Temperatur oberhalb der Schmelztemperatur der nitrierten Produkte gearbeitet wird und/oder daß alle Extraktionsstufen jeweils als Flüssig/Flüssig-Extraktionen durchgeführt werden und/oder daß für den Fall der Aufkonzentrierung die anfallenden aufzukonzentrierenden Lösungen von Salpetersäure, Schwefelsäure und Salpetriger Säure aus allen Extraktionsstufen vereint und gemeinsam aufkonzentriert werden, um nachfolgend der Nitrierung wieder zugeführt zu werden.

14. Verfahren nach einem der Ansprüche 1 bis 13, **dadurch gekennzeichnet, daß** das Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen angewandt wird.

15. Produktionsanlage zur Nitrierung nitrierbarer aromatischer Verbindungen mit nachfolgender Aufreinigung der nitrierten Produkte einschließlich einer Entfernung und Wiedergewinnung von Nitriersäuregemischen, insbesondere Schwefelsäure, Salpetersäure und Stickoxiden,
**dadurch gekennzeichnet,**
**daß** die Produktionsanlage die folgenden Einheiten umfaßt:
a) eine Nitriereinheit (N) zur Nitrierung nitrierbarer aromatischer Verbindungen mit einem oder mehreren entsprechenden Reaktionsbehältern zur Durchführung der Nitrierreaktion(en); und,
b) in Produktionslinie stromabwärts zur Nitriereinheit (N) angeordnet, eine Einheit (WS) zur Durchführung einer sauren Wäsche mittels Extraktion, umfassend:
- eine Querstromextraktionseinheit (WS I) zur Durchführung einer sauren Wäsche nitrierter Rohprodukte mittels Querstromextraktion und,
- in Produktionslinie stromabwärts zur Querstromextraktionseinheit (WS I), eine Gegenstromextraktionseinheit (WS II) zur Durchführung einer sauren Wäsche der zuvor mittels Querstromextraktion gewaschenen nitrierten Rohprodukte mittels Gegenstromextraktion.

## Claims

1. Process for removing and recovering nitrating acid mixtures, in particular nitric acid, sulphuric acid and oxides of nitrogen, from the nitrated crude products occurring in the nitration of nitratable aromatic compounds, after the nitrating acid has been separated off, by a multistage extraction process,
**characterized in that**
the extraction process comprises at least two stages, one stage of which comprises a cross-current extraction and another stage of which comprises a counter-current extraction.

2. Process according to Claim 1, **characterized in that** the counter-current extraction is carried out after the cross-current extraction and/or **in that** the cross-current extraction and/or the counter-current extraction are carried out as a liquid/liquid extraction and/or **in that** the cross-current extraction is carried out in one stage and the counter-current extraction in a plurality of stages.

3. Process according to Claim 2, **characterized in that** the cross-current extraction is carried out in one stage and the counter-current extraction in two to four stages and/or **in that** the cross-current extraction is carried out using an aqueous solution of nitric acid, sulphuric acid and nitrous acid, the volume ratio of the nitrated crude products to the aqueous solution of nitric acid, sulphuric acid and nitrous acid being adjusted to 200 : 1 to 1 : 10.

4. Process according to any of Claims 1 to 3, **characterized in that** the aqueous extract originating from the cross-current extraction is fed back directly or after concentration to the nitration and/or **in that** the acid mixture taken off from the cross-current extraction and comprising sulphuric acid, nitric acid and nitrous acid has a total acid content of 40 to 80 % by weight.

5. Process according to Claim 4, **characterized in that** the aqueous extract originating from the cross-current extraction is fed back directly to the nitration and/or **in that** the acid mixture taken off from the cross-current extraction and comprising sulphuric acid, nitric acid and nitrous acid has a total acid content of 45 to 71 % by weight.

6. Process according to any of Claims 1 to 5, **characterized in that** the counter-current extraction is carried out using an aqueous solution of nitric acid, sulphuric acid and nitrous acid, the volume ratio of the nitrated crude products from the cross-current extraction to the aqueous solution of nitric acid, sulphuric acid and nitrous acid being adjusted to 200 : 1 to 1 : 4, and/or **in that** the adjustment of the volume ratio in the counter-current extraction is effected by addition of water in appropriate amounts to the nitrated crude products and/or by circulation of the aqueous solution of nitric acid, sulphuric acid and nitrous acid.

7. Process according to Claim 6, **characterized in that** the volume ratio of the nitrated crude products from the cross-current extraction to the aqueous solution of nitric acid, sulphuric acid and nitrous acid is adjusted to 50 : 1 to 1 : 2 and/or **in that** the adjustment of the volume ratio in the counter-current extraction is effected by addition of water in amounts of 1 to 10 % by weight, based on the nitrated crude products.

8. Process according to any of Claims 1 to 7, **characterized in that** the acid mixture taken off from the counter-current extraction and comprising sulphuric acid, nitric acid and nitrous acid has a total acid content, calculated as nitric acid, of 15 to 40 % by weight, and/or **in that** the counter-current extraction as such is carried out in a plurality of stages.

9. Process according to Claim 8, **characterized in that** the acid mixture taken off from the counter-current extraction and comprising sulphuric acid, nitric acid and nitrous acid has a total acid content, calculated as nitric acid, of 30 to 35 % by weight and/or **in that** the volume ratio of the nitrated crude products to the aqueous solution of nitric acid, sulphuric acid and nitrous acid is adjusted to 200 : 1 to 1 : 4 in all stages of the counter-current extraction.

10. Process according to any of Claims 1 to 9, **characterized in that** the counter-current extraction is carried out in such a way that the density of the aqueous solution of nitric acid, sulphuric acid and nitrous acid is adjusted in each case to be lower than the density of the nitrated products.

11. Process according to any of Claims 1 to 10, **characterized in that** the aqueous extract originating from the counter-current extraction is fed back directly or after concentration to the nitration, and/or **in that** the nitrated crude products treated by means of counter-current extraction are then fed to a further purification and/or treatment.

12. Process according to Claim 11, **characterized in that,** in the case of concentration, this is carried out to a total acid content of at least 40 % by weight, calculated as nitric acid, and/or **in that** the further purification and/or treatment of the nitrated crude products, treated by means of counter-current extraction, is effected by means of alkaline and subsequent neutral scrubbing, optionally with subsequent drying.

13. Process according to any of Claims 1 to 12, **characterized in that** a temperature above the melting point of the nitrated products is employed in all extraction stages and/or **in that** all extraction stages are carried out in each case as liquid/liquid extractions and/or **in that**, in the case of concentration, the resulting solutions of nitric acid, sulphuric acid and nitrous acid from all extraction stages, which solutions are to be concentrated, are combined and are concentrated together in order to be subsequently recycled to the nitration.

14. Process according to any of Claims 1 to 13, **characterized in that** the process is used for removing and recovering nitric acid, sulphuric acid and oxides of nitrogen from the crude dinitrotoluenes occurring in the nitration of toluene or mononitrotoluenes after the nitrating acid has been separated off.

15. Production plant for nitrating nitratable aromatic compounds with subsequent purification of the nitrated products, including removal and recovery of nitrating acid mixtures, in particular sulphuric acid, nitric acid and oxides of nitrogen,
**characterized in that**
the production plant comprises the following units :
a) a nitrating unit (N) for nitrating nitratable aromatic compounds, having one or more appropriate reaction containers for carrying out the nitration reaction(s); and,
b) arranged in the production line downstream of the nitrating unit (N), a unit (WS) for carrying out acidic scrubbing by means of extraction, comprising :
- a cross-current extraction unit (WS I) for carrying out acidic scrubbing of nitrated crude products by means of cross-current extraction and,
- in the production line downstream of the cross-current extraction unit (WS I), a counter-current extraction unit (WS II) for carrying out acidic scrubbing of the nitrated crude products, scrubbed beforehand by means of cross-current extraction, by means of counter-current extraction.

## Revendications

1. Procédé de séparation et de récupération de mélanges d'acides nitrants, notamment d'acide nitrique, d'acide sulfurique et d'oxydes d'azote, par un procédé d'extraction à plusieurs étapes à partir des produits bruts nitrés formés lors de la nitration de composés aromatiques nitrables après séparation de l'acide nitrant,
**caractérisé en ce que**
le procédé d'extraction comprend au moins deux étapes, parmi lesquelles une étape comprend une extraction à courant transversal et une autre étape comprend une extraction à contre-courant.

2. Procédé selon la revendication 1, **caractérisé en ce que** l'extraction à contre-courant est réalisée à la suite de l'extraction à courant transversal et/ou **en ce que** l'extraction à courant transversal et/ou l'extraction à contre-courant sont réalisées sous la forme d'une extraction liquide/liquide et/ou **en ce que** l'extraction à courant transversal est réalisée en une étape et l'extraction à contre-courant en plusieurs étapes.

3. Procédé selon la revendication 2, **caractérisé en ce que** l'extraction à courant transversal est réalisée en une étape et l'extraction à contre-courant en deux à quatre étapes et/ou **en ce que** l'extraction à courant transversal est réalisée avec une solution aqueuse d'acide nitrique, d'acide sulfurique et d'acide nitreux, le rapport volumique entre les produits bruts nitrés et la solution aqueuse d'acide nitrique, d'acide sulfurique et d'acide nitreux étant ajusté à 200 : 1 à 1 : 10.

4. Procédé selon l'une quelconque des revendications 1 à 3, **caractérisé en ce que** l'extrait aqueux issu de l'extraction à courant transversal est à nouveau introduit dans la nitration, directement ou après une concentration, et/ou **en ce que** le mélange acide d'acide sulfurique, d'acide nitrique et d'acide nitreux extrait de l'extraction à courant transversal présente une teneur totale en acide de 40 à 80 % en poids.

5. Procédé selon la revendication 4, **caractérisé en ce que** l'extrait aqueux issu de l'extraction à courant transversal est à nouveau introduit directement dans la nitration et/ou **en ce que** le mélange acide d'acide sulfurique, d'acide nitrique et d'acide nitreux extrait de l'extraction à courant transversal présente une teneur totale en acide de 45 à 71 % en poids.

6. Procédé selon l'une quelconque des revendications 1 à 5, **caractérisé en ce que** l'extraction à contre-courant est réalisée avec une solution aqueuse d'acide nitrique, d'acide sulfurique et d'acide nitreux, le rapport volumique entre les produits bruts nitrés issus de l'extraction à courant transversal et la solution aqueuse d'acide nitrique, d'acide sulfurique et d'acide nitreux étant ajusté à 200 : 1 à 1 : 4, et/ou **en ce que** l'ajustement du rapport volumique lors de l'extraction à contre-courant a lieu par ajout d'eau en quantités correspondantes aux produits bruts nitrés et/ou par recyclage de la solution aqueuse d'acide nitrique, d'acide sulfurique et d'acide nitreux.

7. Procédé selon la revendication 6, **caractérisé en ce que** le rapport volumique entre les produits bruts nitrés issus de l'extraction à courant transversal et la solution aqueuse d'acide nitrique, d'acide sulfurique et d'acide nitreux est ajusté à 50 : 1 à 1 : 2 et/ou **en ce que** l'ajustement du rapport volumique lors de l'extraction à contre-courant a lieu par ajout d'eau en quantités de 1 à 10 % en poids, par rapport aux produits bruts nitrés.

8. Procédé selon l'une quelconque des revendications 1 à 7, **caractérisé en ce que** le mélange acide d'acide sulfurique, d'acide nitrique et d'acide nitreux extrait de l'extraction à contre-courant présente une teneur totale en acide, calculée en tant qu'acide nitrique, de 15 à 40 % en poids et/ou **en ce que** l'extraction à contre-courant est réalisée en tant que telle en plusieurs étapes.

9. Procédé selon la revendication 8, **caractérisé en ce que** le mélange acide d'acide sulfurique, d'acide nitrique et d'acide nitreux extrait de l'extraction à contre-courant présente une teneur totale en acide, calculée en tant qu'acide nitrique, de 30 à 35 % en poids et/ou **en ce que** dans toutes les étapes de l'extraction à contre-courant, le rapport volumique entre les produits bruts nitrés et la solution aqueuse d'acide nitrique, d'acide sulfurique et d'acide nitreux est ajusté à 200:1 à 1:4.

10. Procédé selon l'une quelconque des revendications 1 à 9, **caractérisé en ce que** l'extraction à contre-courant est réalisée de manière à ce que la densité de la solution aqueuse d'acide nitrique, d'acide sulfurique et d'acide nitreux soit à chaque fois ajustée plus bas que la densité des produits nitrés.

11. Procédé selon l'une quelconque des revendications 1 à 10, **caractérisé en ce que** l'extrait aqueux issu de l'extraction à contre-courant est à nouveau introduit dans la nitration, directement ou après une concentration, et/ou **en ce que** les produits bruts nitrés traités par extraction à contre-courant sont ensuite introduits dans une purification et/ou un conditionnement supplémentaire.

12. Procédé selon la revendication 11, **caractérisé en ce que** dans le cas de la concentration, celle-ci est réalisée jusqu'à une teneur totale en acide d'au moins 40 % en poids, calculée en tant qu'acide nitrique, et/ou **en ce que** la purification et/ou le conditionnement supplémentaire des produits bruts nitrés traités par extraction à contre-courant a lieu par lavage alcalin, puis neutre, éventuellement avec séchage ultérieur.

13. Procédé selon l'une quelconque des revendications 1 à 12, **caractérisé en ce que** les produits nitrés sont traités à une température supérieure à la température de fusion dans toutes les étapes d'extraction et/ou **en ce que** toutes les étapes d'extraction sont à chaque fois réalisées sous la forme d'extractions liquide/liquide et/ou **en ce que** dans le cas de la concentration, les solutions d'acide nitrique, d'acide sulfurique et d'acide nitreux formées à concentrer, issues de toutes les étapes d'extraction, sont réunies et concentrées ensemble, pour être ensuite à nouveau introduites dans la nitration.

14. Procédé selon l'une quelconque des revendications 1 à 13, **caractérisé en ce que** le procédé de séparation et de récupération d'acide nitrique, d'acide sulfurique et d'oxydes d'azote est appliqué aux dinitrotoluènes bruts formés lors de la nitration de toluène ou de mononitrotoluènes après séparation de l'acide nitrant.

15. Installation de production pour la nitration de composés aromatiques nitrables avec purification ultérieure des produits nitrés incluant une séparation et une récupération de mélanges d'acides nitrants, notamment d'acide sulfurique, d'acide nitrique et d'oxydes d'azote,
**caractérisé en ce que**
l'installation de production comprend les unités suivantes :
a) une unité de nitration (N), destinée à la nitration de composés aromatiques nitrables, comprenant un ou plusieurs contenants de réaction correspondants pour la réalisation de la ou des réactions de nitration ; et
b) une unité (WS), placée en aval dans la chaîne de production par rapport à l'unité de nitration (N), destinée à la réalisation d'un lavage acide par extraction, comprenant :
- une unité d'extraction à courant transversal (WS I), destinée à la réalisation d'un lavage acide des produits bruts nitrés par extraction à courant transversal, et
- une unité d'extraction à contre-courant (WS II), placée en aval dans la chaîne de production par rapport à l'unité d'extraction à courant transversal (WS I), destinée à la réalisation d'un lavage acide des produits bruts nitrés lavés au préalable par extraction à courant transversal, par extraction à contre-courant.
